# EUROPÄISCHE PATENTSCHRIFT

(11) **EP 3 302 372 B1**
(45) Veröffentlichungstag und Bekanntmachung des Hinweises auf die Patenterteilung: **24.04.2019**
(21) Anmeldenummer: 15742005.0
(22) Anmeldetag: 23.07.2015
(51) Int. Cl.: A61F 2/915

(54) **STENT**
STENT
STENT

(43) Veröffentlichungstag der Anmeldung: 11.04.2018
(60) Teilanmeldung: 18185569.3
(73) Patentinhaber: optimed medizinische Instrumente GmbH, 76275 Ettlingen (DE)
(72) Erfinder: FISCHER, Harald, 76356 Weingarten (DE); NENNIG, Ernst, 76131 Karlsruhe (DE)
(74) Vertreter: Manitz Finsterwald Patent- und Rechtsanwaltspartnerschaft mbB
(86) Internationale Anmeldenummer: PCT/EP2015/066895
(87) Internationale Veröffentlichungsnummer: WO 2017/012673

(56) Entgegenhaltungen:
- EP-A1- 2 561 837
- EP-A1- 2 594 232
- WO-A1-2014/205124
- US-A1- 2004 034 402
- US-A1- 2004 044 401
- US-A1- 2010 152 835
- US-A1- 2012 245 671

## Beschreibung

Die vorliegende Erfindung betrifft einen Stent zur transluminalen Implantation in Hohlorgane, insbesondere in Blutgefäße, Harnleiter, Speiseröhren, Colon, Duodenum oder Gallenwege, mit einem im Wesentlichen röhrenförmigen Körper, der von einem komprimierten Zustand mit einem ersten Querschnittsdurchmesser in einen expandierten Zustand mit einem vergrößerten zweiten Querschnittsdurchmesser überführbar ist, wobei der Stent eine Vielzahl von Zellen umfasst, welche von durch den röhrenförmigen Körper gebildeten Umrandungselementen definiert werden.

Stents dieser Art werden zur Rekanalisation von krankhaft veränderten Hohlorganen eingesetzt. Dabei werden die Stents im komprimierten Zustand über einen Einführkatheter an die zu behandelnde Stelle innerhalb des Hohlorgans eingeführt, wo sie durch unterschiedliche Maßnahmen auf einen Durchmesser, der dem Durchmesser des gesunden Hohlorgans entspricht, expandiert werden, so dass eine Stützwirkung des Hohlorgans, beispielsweise einer Gefäßwand, erreicht wird.

Derartige Stents können zum Beispiel dadurch erzeugt werden, dass in die Wand eines röhrenförmigen Körpers Durchbrechungen wie zum Beispiel Schlitze geschnitten werden, die sich teilweise in Längsrichtung des Stents erstrecken, so dass bei der Expansion des Stents beispielsweise rautenförmige Durchbrechungen entstehen. Eine Durchbrechung zusammen mit ihren Umrandungselementen wird als Zelle bezeichnet.

Werden Stents in der Nähe einer Bifurkation eines Hohlorgans eingesetzt, so können Stents verwendet werden, die ein abgeschrägtes Ende aufweisen. Solche Stents bieten die Möglichkeit, z.B. eine Vene allseitig bis zur Bifurkation abzustützen, d.h. beispielsweise bis zur Mündung in eine weitere Vene.

Um ihre Stützwirkung gewährleisten zu können, müssen die Stents in der Lage sein, eine ausreichende radiale Aufstellkraft auszuüben, die einer von der Gefäßwand ausgeübten radialen Krafteinwirkung entgegenwirkt. Dies gilt besonders im Bereich des abgeschrägten Endes, da dort die radiale Aufstellkraft üblicherweise reduziert ist.

Die US 2012/024567 A1 beschreibt einen mit einem im Wesentlichen röhrenförmigen Körper, der von einem komprimierten Zustand mit einem ersten Querschnittsdurchmesser in einen expandierten Zustand mit einem vergrößerten zweiten Querschnittsdurchmesser überführbar ist. Der Stent umfasst eine Vielzahl von Zellen, die von durch den röhrenförmigen Körper gebildeten Umrandungselementen definiert werden. Ein Teil der Zellen ist im Vergleich zu den übrigen Zellen in Längsrichtung des Stents verlängert ausgebildet.

Die US 2012/24567 A1 beschreibt einen Stent, der aus einem rohrförmigen Material geschnitten und bis zu einem expandierten Zustand aufgeweitet wird.

Es ist die Aufgabe der vorliegenden Erfindung, einen Stent der eingangs genannten Art zu schaffen, der auch in einem abgeschrägten Bereich eine hohe radiale Aufstellkraft bereitstellt, wodurch ein Einknicken des Stents bei dessen Einsatz zuverlässig ausgeschlossen wird.

Diese Aufgabe wird erfindungsgemäß durch einen Stent mit den Merkmalen des Anspruchs 1 und insbesondere dadurch gelöst, dass ein Teil der Zellen im Vergleich zu den übrigen Zellen in Längsrichtung des Stents verlängert ausgebildet ist, um ein abgeschrägtes stirnseitiges Ende des Stents zu bilden.

Aufgrund der verlängerten Zellen kann ein abgeschrägtes Ende erzeugt werden. Dabei werden wegen der in Längsrichtung verlängerten Zellen keine zusätzlichen Zellen benötigt, um das abgeschrägte Ende zu bilden. Mittels der verlängerten Zellen wird es ermöglicht, auch in dem abgeschrägten Bereich eine ähnliche Anordnung der Zellen zu wählen, wie in dem übrigen röhrenförmigen Körper.

Die verlängerten Zellen können insbesondere nur in einem starren Abschnitt des Stents vorliegen. Zusätzlich kann der Stent z.B. einen flexiblen Abschnitt und/oder einen Verankerungsabschnitt umfassen. Die nachfolgenden Ausführungen bezüglich der verlängerten Zellen beziehen sich auf den starren Abschnitt.

Durch die Vermeidung zusätzlicher Zellen ergibt sich eine Struktur des Stents, die eine besonders hohe radiale Aufstellkraft bereitstellen kann. Auf diese Weise wird es ermöglicht, beispielsweise Blutgefäße in der Nähe von Bifurkationen zuverlässig abzustützen. Ein erfindungsgemäßer Stent kann deshalb beispielsweise bei venösen Obstruktionen im Bereich der Bifurkation, dem Zusammenfluss der beiden Venae iliaca communis in die Vena cava inferior, im oberen Bereich der Vena iliaca communis eingesetzt werden. Der Stent kann dazu einen Durchmesser von größer oder gleich 12 mm aufweisen. Bevorzugt kann der Stent einen Durchmesser zwischen 12 mm und 18 mm besitzen.

Weiterhin erlaubt es der Verzicht auf zusätzliche Zellen, den Winkel der Abschrägung variabel festzulegen, da dieser Winkel durch die relative Verlängerung der verlängerten Zellen festgelegt werden kann.

Generell erlaubt es der abgeschrägte Bereich, das Hohlorgan bis zur Bifurkation zuverlässig abzustützen, ohne jedoch z.B. wesentlich in die Blutbahn nach der Bifurkation hineinzuragen.

Eine Zelle kann durch einen Verbindungsabschnitt oder mehrere Verbindungsabschnitte mit einer oder mehreren anderen Zellen verbunden sein. Unter der Länge einer Zelle kann der Abstand in Längsrichtung zwischen zwei Verbindungsabschnitten verstanden werden, wobei jeweils die Mitte des jeweiligen Verbindungsabschnitts zu berücksichtigen ist. Eine Zelle umfasst die genannte Aussparung sowie ihre jeweiligen Umrandungselemente, wobei die Verbindungsabschnitte zu den Umrandungselementen gehören.

Bei einem erfindungsgemäßen Stent können die Zellen zumindest zum Teil jeweils mittels mehreren Verbindungsabschnitten miteinander verbunden sein. Insbesondere können in dem abgeschrägten Bereich und/oder bei den verlängerten Zellen jeweils drei oder vier Verbindungsabschnitte vorgesehen sein. Somit kann die Stützwirkung durch die auf diese Weise zu erreichende radiale Aufstellkraft besonders hoch sein.

Der Stent kann aus einem Memory-Metall gefertigt sein, das ab einer Grenztemperatur eine gespeicherte Form einnimmt.

Bevorzugte Ausführungsformen der Erfindung sind der Beschreibung, den Unteransprüchen und den Zeichnungen zu entnehmen.

Gemäß einer ersten vorteilhaften Ausführungsform ist zumindest ein Teil der verlängerten Zellen entlang einer geraden oder annähernd geraden Linie angeordnet, die insbesondere parallel oder annähernd parallel zu der Längsrichtung verläuft. Dies bedeutet, dass beispielsweise zumindest eine oder zwei auf der geraden Linie hintereinander angeordnete Zellen vorgesehen sein können. Bei mehr als zwei Zellen können zumindest zwei Verbindungsabschnitte zumindest einer dieser Zellen mit zwei weiteren der verlängerten Zellen direkt verbunden sein. Insbesondere liegen jeweils zwei Verbindungsabschnitte der verlängerten Zellen auf der geraden Linie.

Gemäß einer weiteren vorteilhaften Ausführungsform sind die verlängerten Zellen in mehrere Gruppen, insbesondere in neun Gruppen, einteilbar, wobei die Zellen jeder Gruppe jeweils entlang einer geraden oder annähernd geraden Linie angeordnet sind und diese Linien insbesondere parallel oder annähernd parallel zu der Längsrichtung verlaufen. Insgesamt können bei dem Stent zwölf Gruppen von Zellen vorgesehen sein, von welchen neun Gruppen verlängerte Zellen aufweisen. Bei einer solchen Definition von Gruppen können die Zwischenräume zwischen den Gruppen selbst auch Zellen bilden.

Die Anordnung der Zellen kann also derart gewählt sein, dass die jeweiligen Zellen entlang von geraden oder annähernd geraden Linien angeordnet sind. Zusätzlich können die Zellen symmetrisch zu einer dieser Linien ausgebildet sein. Es können also insbesondere keine Zellen vorliegen, die geneigt bzw. gedreht zu den übrigen Zellen sind. Auf diese Weise kann eine Schwächung der Struktur durch solche gedrehten Zellen vermieden werden.

Insbesondere können alle Zellen innerhalb einer Gruppe in Längsrichtung gesehen jeweils die gleiche oder die annähernd gleiche Länge aufweisen. Alternativ können in einer Gruppe auch Zellen mit unterschiedlichen Längen vorgesehen sein.

Bevorzugt verlaufen die Linien, welche durch die Zellen verschiedener Gruppen gebildet werden, parallel oder annähernd parallel zueinander.

Weiter bevorzugt sind von einer sich senkrecht zur Längsachse erstreckenden Querschnittsebene bis zu dem abgeschrägten stirnseitigen Ende jeweils gleich viele Zellen in jeder Gruppe vorgesehen. Durch die Vorsehung jeweils gleich vieler Zellen in einer Gruppe kann eine radiale Aufstellkraft bewirkt werden, welche über die Länge des Stents im Wesentlichen konstant ist. Bevorzugt können in dem starren Abschnitt des Stents in jeder Gruppe gleich viele Zellen vorgesehen sein.

Insbesondere bei der Verwendung gleich vieler Zellen können von den Verbindungsabschnitten der Zellen jeweils Winkel gebildet werden, die in der sogenannten Abwicklung des Stents zu erkennen sind. Die Winkel werden durch die Umfangsrichtung (welche in der Abwicklung eine Gerade ist) und eine gerade Linie definiert, wobei die gerade Linie durch Verbindungsabschnitte der Zellen verläuft. Dies bedeutet, dass die Verbindungsabschnitte jeweils zumindest eines Teils der Zellen in der Abwicklung auf geraden Linien liegen. Die Winkel können umso größer sein, je näher der Winkel dem abgeschrägten Ende des Stents ist, wobei bevorzugt vier, fünf oder sechs Winkel vorgesehen sind.

An dem abgeschrägten Ende selbst können die Enden der Zellen in der Abwicklung keine gerade Linie bilden, sondern eine Kurve definieren, welche einer Sinus-Kurve angenähert ist. Eine solche Sinus-Kurve in der Abwicklung führt am dreidimensionalen Stent zu einem schrägen Anschnitt (dem abgeschrägten Bereich) mit einer exakt ebenen Schnittfläche.

Die Zellen können so angeordnet sein, dass ein erster Winkel in einem Bereich zwischen 20° und 24°, ein zweiter Winkel in einem Bereich zwischen 37° und 44°, ein dritter Winkel in einem Bereich zwischen 48° und 52°, ein vierter Winkel in einem Bereich zwischen 60° und 64°, ein fünfter Winkel in einem Bereich zwischen 63° und 67° und ein sechster Winkel in einem Bereich zwischen 69° und 73° liegt. Der größte Winkel kann insbesondere am nächsten an dem abgeschrägten Ende des Stents liegen, wohingegen der kleinste Winkel am weitesten entfernt von dem abgeschrägten Ende liegt. Zudem kann ein Winkel von 0° vorgesehen sein, dies bedeutet, dass eine Position an dem Stent in der Abwicklung existiert, an welcher Verbindungsabschnitte entlang der Umfangsrichtung angeordnet sind. Der Winkel von 0° kann an einem Übergang von dem starren Bereich zu dem flexiblen Bereich vorgesehen sein. Die Zellen einer Gruppe können unterschiedlich lang ausgeführt sein, um die beschriebenen Winkel zu bilden.

Für eine derartige Auswahl der Winkel hat es sich gezeigt, dass auf diese Weise ein sehr stabiler Stent geschaffen werden kann, der eine besonders lange Haltbarkeit aufweist.

Gemäß einer weiteren vorteilhaften Ausführungsform fällt die Länge der Zellen benachbarter Gruppen in Umfangsrichtung von einem Maximum bis zu einem Minimum. Insbesondere liegen Zellen mit maximaler Länge Zellen mit minimaler Länge bezüglich einer zentralen Achse des Stents gegenüber. Durch eine derartige Anordnung kann die Abschrägung erzeugt werden.

Weiter bevorzugt sind die Zellen zumindest zum Teil mittels Verbindungsabschnitten miteinander verbunden, wobei die Verbindungsabschnitte zwischen den verlängerten Zellen, insbesondere nur zwischen den längsten Zellen, verlängert ausgebildet sind. Aufgrund der verlängerten bzw. vergrößerten Verbindungsabschnitte können die Öffnungen der längsten Zellen verkürzt werden, wodurch beim Expandieren des Stents ein gleichmäßiges Aufbiegen sämtlicher Zellen erreicht werden kann. Ein derart gleichmäßiges Aufbiegen kann wiederum zu einer gleichmäßigen Verteilung der radialen Aufstellkraft sowie zu einem besonders robusten Stent führen.

Besonders bevorzugt erstreckt sich in Längsrichtung von dem abgeschrägten Ende zumindest ein Marker weg, insbesondere in Form einer Öse, wobei der Marker eine asymmetrische Form aufweist. Ein Marker kann ein Abschnitt des Stents sein, der eine erhöhte Röntgendichtigkeit aufweist, d.h. in einem Röntgenbild besonders gut sichtbar ist. Insbesondere kann es sich bei dem Marker um eine Öse handeln, die beispielsweise mit Tantal gefüllt oder bedeckt ist. Aufgrund der asymmetrischen Form kann der Marker auch in dem abgeschrägten Bereich angebracht sein, da sich der Marker von der Abschrägung weg erstrecken kann.

Anders ausgedrückt kann der Marker also in einem Bereich zwischen der längsten und der kürzesten Erstreckung des Stents in Längsrichtung angeordnet sein. Aufgrund der asymmetrischen Form kann der Marker groß genug ausgeführt werden, um besonders gut im Röntgenbild erkannt zu werden. Zusätzlich kann ein weiterer Marker z.B. an der Spitze der Abschrägung vorgesehen sein. Noch ein weiterer Marker kann beispielsweise an einer kürzesten Stelle des Stents an der Abschrägung angebracht sein.

Gemäß einer weiteren vorteilhaften Ausführungsform sind zumindest zwei asymmetrische Marker an dem abgeschrägten Ende vorgesehen, wobei sich die Marker bezüglich einer Achse des Stents insbesondere gegenüberliegen. Die beiden Marker können also symmetrisch zu einer Ebene des Stents liegen, welche durch eine Achse des Stents und eine Spitze der Abschrägung verläuft. Aufgrund einer solchen Anordnung können die asymmetrischen Marker in einem Röntgenbild beispielsweise zur Deckung gebracht werden. In der Folge kann die Lage des Stents im Röntgenbild besonders gut zu erkennen sein.

Gemäß einer weiteren vorteilhaften Ausführungsform umfasst der Stent einen flexiblen Abschnitt, der sich an den starren Abschnitt anschließt. Der flexible Abschnitt liegt dem abgeschrägten Ende gegenüber. Der flexible Abschnitt kann Zellen aufweisen, welche in der Abwicklung eine größere Fläche aufweisen als Zellen des starren Abschnitts. Aufgrund der größeren Zellen kann der flexible Abschnitt leichter biegbar sein, wodurch sich der flexible Abschnitt auf einfache Weise an die Verlaufsform eines Hohlorgans anpassen kann. Bevorzugt können die Zellen des flexiblen Abschnitts eine zahnförmige Begrenzung aufweisen.

Gemäß noch einer weiteren vorteilhaften Ausführungsform umfasst der Stent einen Verankerungsabschnitt, der sich an den flexiblen Abschnitt anschließt. Die Zellen des Verankerungsabschnitts können den Zellen des starren Abschnitts entsprechen und beispielsweise rautenförmig ausgebildet sein. Der Verankerungsabschnitt kann aufgrund der rautenförmigen Zellen eine geringe Flexibilität aufweisen und so den Stent an seiner Position in dem Hohlorgan fixieren. Der Verankerungsabschnitt kann ein gerades Ende des Stents bilden, an welchem Marker angebracht sein können, die sich von einem Ende des Stents weg erstrecken. Die Marker können die Form von Ösen haben und ebenfalls z.B. mit Tantal bedeckt oder gefüllt sein. Beim Einführen des Stents in das Hohlorgan kann der Stent mittels der Marker des Verankerungsabschnitts in einem Einführkatheter fixiert werden.

Der beschriebene Stent kann in einem Verfahren verwendet werden, bei welchem
a) der Stent aus einem röhrenförmigen Material geschnitten wird,
b) der Stent bis zu seinem expandierten Zustand aufgeweitet wird, und
c) in dem expandierten Zustand die Form der Zellen des Stents geändert und fixiert wird.

Durch die Änderung der Form der Zellen können die einzelnen Zellen jeweils derart geformt werden, dass ein gleichmäßiges Aufdehnverhalten der einzelnen Zellen erreicht wird. Auf diese Weise kann die Bruchgefahr insbesondere bei kurzen und mittleren Zellen in der Nähe des abgeschrägten Bereichs reduziert werden, welche sich üblicherweise aufgrund einer inhomogenen Kraftverteilung beim Expandieren unsymmetrisch und übermäßig aufdehnen.

Beispielsweise können bei rautenförmigen Zellen mit bis zu vier Verbindungsabschnitten an den jeweiligen Ecken der Raute die spitzen Winkel derart verringert oder verändert werden, dass diese kleiner als 70°, bevorzugt kleiner als 60°, sind. Dies führt dazu, dass der Stent großen äußeren Kräften im Bereich des abgeschrägten Bereichs durch eine homogene Kraftableitung über die Struktur besser standhalten kann und das Risiko eines Stentkollapses oder eines Stentbruchs deutlich verringert wird.

Mittels des Verfahrens ist es zudem möglich, ein zu weites Aufdehnen von Zellen zu verhindern, wodurch eine Vorschädigung von Verbindungsabschnitten vermieden werden kann.

Zum Aufweiten kann ein Kern verwendet werden, an welchem Befestigungsmittel angebracht werden, um die Form der Zellen des Stents zu ändern und zu fixieren. Mit den Befestigungsmitteln kann also das Aufdehnverhalten der einzelnen Zellen angepasst werden. Die Form der Zellen wird folglich gegenüber der Aufdehnung bei der reinen Verwendung z.B. eines zylinderförmigen Kerns verändert. Ein solcher zylinderförmiger Kern kann auch einen kegelförmigen Abschnitt umfassen, der das Aufziehen des Stents erleichtert. Zudem kann das Aufdehnen unter Wärmezuführung erfolgen.

Die Befestigungsmittel können Nadeln oder Dorne sein, die in Löcher des Kerns eingebracht werden. Die Nadeln oder Dorne können beispielsweise von innen aus dem Kern herausgefahren oder von außen in den Kern eingesteckt werden. Hierzu kann beispielsweise ein automatisierter Prozess mittels Robotern oder einer Hydraulik aber auch eine manuelle Anpassung der Zellen durchgeführt werden.

Die geänderte Form der Zellen des Stents kann mittels eines Wärmeprozesses dauerhaft fixiert werden. Eine solche Fixierung ist insbesondere bei der Verwendung von Memory-Metallen von Vorteil, die bei Temperaturerhöhung die durch den Wärmeprozess gespeicherte Form wieder einnehmen. Unter einer dauerhaften Fixierung ist eine Fixierung der Form der Zellen des Stent im expandierten Zustand des Stents zu verstehen, wobei die Form der Zellen im expandierten Zustand beibehalten wird, auch wenn der Stent zwischenzeitlich in den komprimierten Zustand überführt wird. Beim Einbringen des Stents in den Körper kann dann durch die Körperwärme der Stent und dessen Zellen die während des Herstellungsprozesses eingelernte Form wieder annehmen.

Nachfolgend wird die Erfindung anhand vorteilhafter Ausführungsformen unter Bezugnahme auf die beigefügten Zeichnungen beschrieben. Es zeigen:
- Fig. 1: einen erfindungsgemäßen Stent im expandierten Zustand in Seitenansicht;
- Fig. 2: den Stent von Figur 1 im expandierten Zustand in einer Draufsicht;
- Fig. 3: den Stent von Figur 1 in einer in eine Ebene projizierten Schneiddarstellung; und
- Fig. 4: die Schneiddarstellung von Fig. 3 mit einer Darstellung von Winkeln, welche durch Verbindungsabschnitte definiert werden.

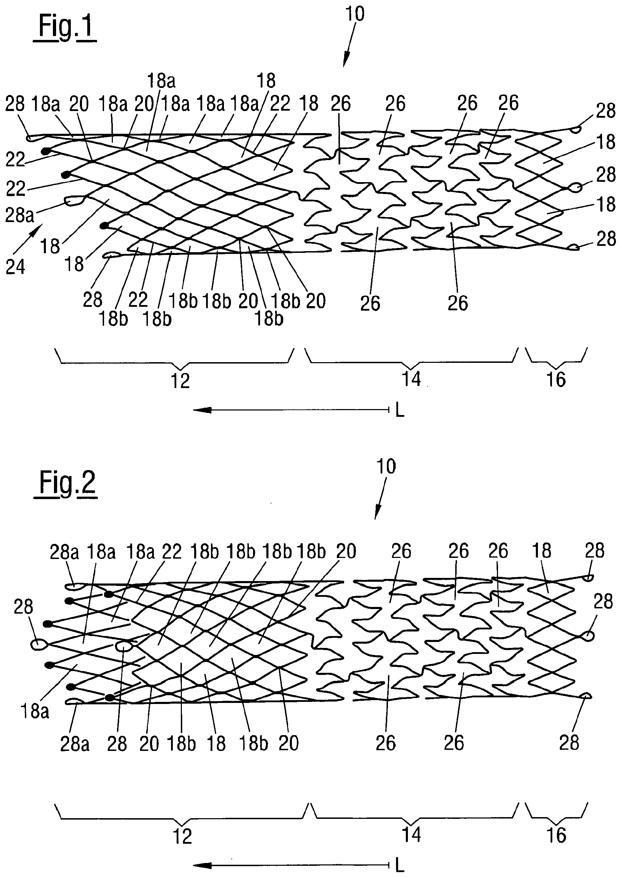

Fig. 1 und Fig. 2 zeigen einen Stent 10. Der Stent 10 weist eine röhrenförmige Gestalt auf und umfasst einen starren Abschnitt 12, einen an den starren Abschnitt 12 anschließenden flexiblen Abschnitt 14 und einen an den flexiblen Abschnitt 14 anschließenden Verankerungsabschnitt 16.

Der starre Abschnitt 12 ist aus rautenförmigen (geschlossenen) Zellen 18 gebildet, welche jeweils über drei oder vier Verbindungsabschnitte 20 mit anderen rautenförmigen Zellen 18 verbunden sind. Die rautenförmigen Zellen 18 werden durch stegartige Umrandungselemente 22 definiert, die aus einem Metall geformt sind.

Der starre Abschnitt 12 umfasst einen abgeschrägten Bereich 24, welcher den Einsatz des Stents 10 an einer (nicht gezeigten) Bifurkation eines Hohlorgans ermöglicht.

Der abgeschrägte Bereich 24 bildet ein Ende des Stents 10 und wird erzeugt, indem ein Teil der rautenförmigen Zellen 18 in einer Längsrichtung L verlängert ausgebildet ist. Die längsten rautenförmigen Zellen 18 sind in den Figuren mit dem Bezugszeichen 18a gekennzeichnet, wohingegen die kürzesten rautenförmigen Zellen 18 mit dem Bezugszeichen 18b gekennzeichnet sind. In dem starren Abschnitt 12 sind jeweils drei der kürzesten rautenförmigen Zellen 18b und drei der längsten rautenförmigen Zellen 18a in Längsrichtung L vorgesehen. Die längsten rautenförmigen Zellen 18a liegen dabei den kürzen rautenförmigen Zellen 18b bezüglich einer zentralen Achse des Stents 10 gegenüber. Es sind jeweils drei Gruppen der längsten und der kürzesten rautenförmigen Zellen 18a, 18b nebeneinander (d.h. in Umfangsrichtung benachbart) vorgesehen.

In dem flexiblen Abschnitt 14 sind offene Zellen 26 mit gezacktem bzw. zahnförmigem Umriss angeordnet, wobei in Umfangsrichtung des Stents 10 gesehen jeweils weniger offene gezackte Zellen 26 vorgesehen sind als rautenförmige Zellen 18. Durch die Verwendung weniger offener gezackter Zellen 26 ist der flexible Abschnitt leichter bezüglich der Längsrichtung L verformbar und kann sich so dem Verlauf einer Blutbahn oder ähnlichem gut anpassen.

Der Verankerungsabschnitt 16 ist durch rautenförmige Zellen 18 gebildet, die für eine erhöhte Steifigkeit des Verankerungsabschnitts 16 sorgen, wodurch der Stent 10 seine Position in einem Hohlorgan zuverlässig beibehält.

Sowohl an dem abgeschrägten Bereich 24 als auch an dem durch den Verankerungsabschnitt gebildeten Ende des Stents 10 sind jeweils vier ösenförmige Marker 28 vorgesehen, von welchen in Fig. 1 jeweils drei sichtbar sind. In Fig. 2 sind alle vier Marker 28 des abgeschrägten Bereichs 24 zu erkennen.

Zwei der Marker 28, welche in dem abgeschrägten Bereich 24 an den Stellen der längsten und der kürzesten Erstreckung des Stents 10 angebracht sind, sind symmetrisch ausgebildet. Zwei weitere Marker 28 sind dort an dem abgeschrägten Bereich 24 angebracht, wo der Stent 10 seine durchschnittliche Länge aufweist. Diese beiden Marker 28 sind als asymmetrische Marker 28a ausgebildet, wobei sich die Fläche der asymmetrischen Marker 28a zu der kürzesten Erstreckung des Stents hin erstreckt.

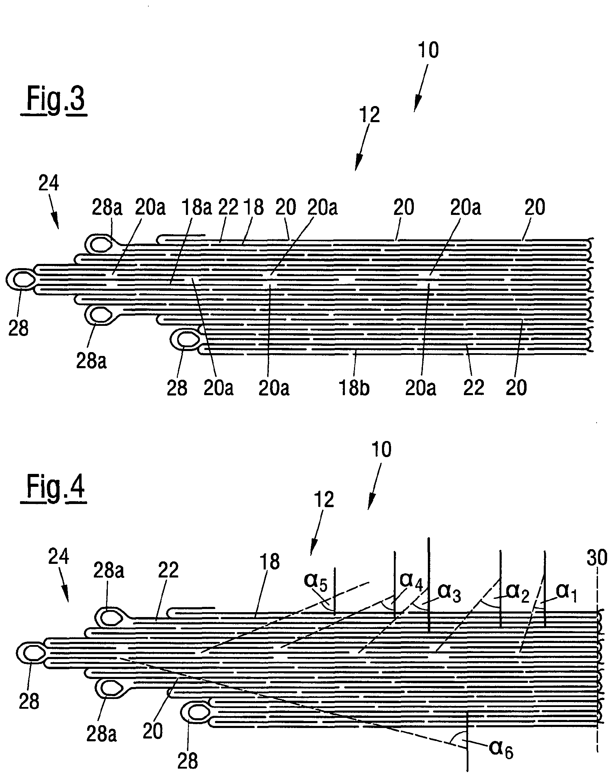

Fig. 3 zeigt den starren Abschnitt 12 des Stents 10 von Fig. 1 und Fig. 2 in einer sogenannten Schneiddarstellung. Fig. 3 zeigt folglich eine Projektion von Schnitten, die in ein Rohmaterial des Stents eingebracht werden, in eine Ebene. Eine Linie zeigt somit einen Schnitt an. Mehrere versetzt zueinander parallel verlaufende gerade Schnitte können beim Expandieren des Stents 10 zu den in Fig. 1 und Fig. 2 gezeigten rautenförmigen Zellen 18 aufgeweitet werden.

Die als weiße Bereiche dargestellten, zwischen den Linien vorhandenen Materialbereiche werden nach dem Expandieren zu Verbindungsabschnitten 20 oder Umrandungselementen 22. Fig. 3 zeigt nur den starren Abschnitt 12 des Stents 10.

In Fig. 3 ist zu erkennen, dass zwischen den längsten rautenförmigen Zellen 18a verlängerte Verbindungsabschnitte 20a vorgesehen sind, die zu einem gleichmäßigeren Aufbiegen aller rautenförmigen Zellen 18 beim Expandieren des Stents führen.

Fig. 4 zeigt die Ansicht von Fig. 3 mit eingetragenen Winkeln, welche von Verbindungsabschnitten 20 mit einer Umfangsrichtung gebildet werden. Gezeigt sind sechs Winkel α₁, α₂, α₃, α₄, α₅, α₆, die von einem Winkel von etwa 22° (α₁) über Winkel von etwa 40° (α₂), 50° (α₃), 62° (α₄) und 65° (α₅) bis zu einem Winkel von etwa 71° (α₆) stetig zunehmen. Eine gerade Endlinie 30, welche an dem Übergang von dem starren Bereich 12 zu dem flexiblen Bereich 14 angeordnet ist, erstreckt sich in Umfangsrichtung durch Verbindungsabschnitte 20 und definiert somit einen Winkel von 0°.

### Bezugszeichenliste

- 10: Stent
- 12: starrer Abschnitt
- 14: flexibler Abschnitt
- 16: Verankerungsabschnitt
- 18, 18a, 18b: rautenförmige Zelle
- 20, 20a: Verbindungsabschnitt
- 22: Umrandungselement
- 24: abgeschrägter Bereich
- 26: offene gezackte Zelle
- 28, 28a: Marker
- 30: Endlinie

- L: Längsrichtung
- α: Winkel

## Patentansprüche

1. Stent (10) zur transluminalen Implantation in Hohlorgane, insbesondere in Blutgefäße, Harnleiter, Speiseröhren, Colon, Duodenum oder Gallenwege, mit einem im Wesentlichen röhrenförmigen Körper, der von einem komprimierten Zustand mit einem ersten Querschnittsdurchmesser in einen expandierten Zustand mit einem vergrößerten zweiten Querschnittsdurchmesser überführbar ist, wobei
der Stent (10) eine Vielzahl von Zellen (18, 18a, 18b) umfasst, welche von durch den röhrenförmigen Körper gebildeten Umrandungselementen (22) definiert werden,
wobei ein Teil der Zellen (18, 18a) im Vergleich zu den übrigen Zellen (18b) in Längsrichtung des Stents (10) verlängert ausgebildet ist, um ein abgeschrägtes stirnseitiges Ende (24) des Stents (10) zu bilden,
**dadurch gekennzeichnet, dass**
zumindest ein Teil der verlängerten Zellen (18, 18a) entlang einer geraden oder annähernd geraden Linie angeordnet ist, die parallel oder annähernd parallel zu der Längsrichtung (L) verläuft.

2. Stent (10) nach Anspruch 1,
**dadurch gekennzeichnet, dass**
die verlängerten Zellen (18, 18a) in mehrere Gruppen, insbesondere in neun Gruppen, einteilbar sind, wobei die Zellen (18, 18a) jeder Gruppe jeweils entlang einer geraden oder annähernd geraden Linie angeordnet sind, wobei die Linien insbesondere parallel oder annähernd parallel zu der Längsrichtung (L) verlaufen.

3. Stent (10) nach Anspruch 2,
**dadurch gekennzeichnet, dass**
die Linien parallel oder annähernd parallel zueinander verlaufen.

4. Stent (10) nach Anspruch 2 oder 3,
**dadurch gekennzeichnet, dass**
von einer sich senkrecht zur Längsachse erstreckenden Querschnittsebene bis zu dem abgeschrägten stirnseitigen Ende (24) jeweils gleich viele Zellen (18, 18a, 18b) in jeder Gruppe vorgesehen sind.

5. Stent (10) nach zumindest einem der Ansprüche 2 bis 4,
**dadurch gekennzeichnet, dass**
die Länge der Zellen (18, 18a, 18b) benachbarter Gruppen in Umfangsrichtung von einem Maximum bis zu einem Minimum fällt.

6. Stent (10) nach zumindest einem der vorstehenden Ansprüche,
**dadurch gekennzeichnet, dass**
Zellen mit maximaler Länge (18a) Zellen mit minimaler Länge (18b) bezüglich einer zentralen Achse des Stents (10) gegenüberliegen.

7. Stent (10) nach zumindest einem der vorstehenden Ansprüche,
**dadurch gekennzeichnet, dass**
die Zellen (18, 18a, 18b) zumindest zum Teil mittels Verbindungsabschnitten (20, 20a) miteinander verbunden sind und die Verbindungsabschnitte (20a) zwischen den verlängerten Zellen (18, 18a), insbesondere nur zwischen den längsten Zellen (18a), verlängert ausgebildet sind.

8. Stent (10) nach zumindest einem der vorstehenden Ansprüche,
**dadurch gekennzeichnet, dass**
in Längsrichtung (L) von dem abgeschrägten Ende (24) sich zumindest ein Marker (28, 28a), insbesondere in Form einer Öse, weg erstreckt, wobei der Marker (28, 28a) eine asymmetrische Form aufweist.

9. Stent (10) nach Anspruch 8,
**dadurch gekennzeichnet, dass**
der Marker (28, 28a) in einem Bereich zwischen der längsten und der kürzesten Erstreckung des Stents (10) in Längsrichtung (L) angeordnet ist.

10. Stent (10) nach Anspruch 8 oder 9,
**dadurch gekennzeichnet, dass**
zumindest zwei Marker (28, 28a) an dem abgeschrägten Ende vorgesehen sind, wobei sich die Marker (28, 28a) bezüglich einer Achse des Stents (10) gegenüberliegen.

11. Stent (10) nach Anspruch 1, der aus einem röhrenförmigen Material geschnitten ist und bei dem im expandiertem Zustand die Form der Zellen (18, 18a, 18b) fixierbar ist.

12. Stent gemäß Anspruch 11,
**dadurch gekennzeichnet, dass**
die Zellen (18, 18a, 18b) des Stents (10) in geänderte Form mittels eines Wärmeprozesses dauerhaft fixierbar sind.

## Claims

1. A stent (10) for transluminal implantation into hollow organs, in particular into blood vessels, ureters, esophagi, the colon, the duodenum or the biliary tract, having a substantially tubular body which can be converted from a compressed state having a first cross-sectional diameter into an expanded state having an enlarged second cross-sectional diameter, wherein
the stent (10) comprises a plurality of cells (18, 18a, 18b) which are defined by bordering elements (22) formed by the tubular body,
wherein some of the cells (18, 18a) are formed as elongated in the longitudinal direction of the stent (10) in comparison with the other cells (18b) to form a chamfered front face end (24) of the stent (10), **characterized in that**
at least some of the elongated cells (18, 18a) are arranged along a straight line or an approximately straight line which extends in parallel or approximately in parallel with the longitudinal direction (L).

2. A stent (10) in accordance with claim 1,
**characterized in that**
the elongated cells (18, 18a) can be divided into a plurality of groups, in particular into nine groups, with the cells (18, 18a) of each group being respectively arranged along a straight line or an approximately straight line, with the lines in particular extending in parallel or approximately in parallel with the longitudinal direction (L).

3. A stent (10) in accordance with claim 2,
**characterized in that**
the lines extend in parallel or approximately in parallel with one another.

4. A stent (10) in accordance with claim 2 or claim 3,
**characterized in that**
a respective equal number of cells (18, 18a, 18b) is provided in each group from a cross-sectional plane extending perpendicular to the longitudinal axis up to the chamfered front face end (24).

5. A stent (10) in accordance with at least one of the claims 2 to 4,
**characterized in that**
the length of the cells (18, 18a, 18b) of adjacent groups decreases from a maximum to a minimum in the peripheral direction.

6. A stent (10) in accordance with at least one of the preceding claims,
**characterized in that**
cells having a maximum length (18a) are disposed opposite cells having a minimal length (18b) with respect to a central axis of the stent (10).

7. A stent (10) in accordance with at least one of the preceding claims,
**characterized in that**
at least some of the cells (18, 18a, 18b) are connected to one another by means of connection sections (20, 20a); and **in that** the connection sections (20a) are formed as elongated between the elongated cells (18, 18a), in particular only between the longest cells (18a).

8. A stent (10) in accordance with at least one of the preceding claims,
**characterized in that**
at least one marker (28, 28a), in particular in the form of an eyelet, extends away from the chamfered end (24) in the longitudinal direction (L), with the marker (28, 28a) having an asymmetrical shape.

9. A stent (10) in accordance with claim 8,
**characterized in that**
the marker (28, 28a) is arranged in a region between the longest and the shortest extent of the stent (10) in the longitudinal direction (L).

10. A stent (10) in accordance with claim 8 or claim 9,
**characterized in that**
at least two markers (28, 28a) are provided at the chamfered end, with the markers (28, 28a) being disposed opposite one another with respect to an axis of the stent (10).

11. A stent (10) in accordance with claim 1 which is cut out of a tubular material and in which the shape of the cells (18, 18a, 18b) can be fixed in the expanded state.

12. A stent in accordance with claim 11,
**characterized in that**
the cells (18, 18a, 18b) of the stent (10) can be permanently fixed in a changed shape by means of a heating process.

## Revendications

1. Stent (10) destiné à l'implantation transluminale dans des organes creux, en particulier dans des vaisseaux sanguins, des uretères, des oesophages, dans le côlon, le duodénum ou dans des voies biliaires, comportant un corps sensiblement tubulaire qui est transférable d'un état comprimé ayant un premier diamètre de section transversale vers un état expansé ayant un second diamètre de section transversale agrandi,
dans lequel
le stent (10) comprend une multitude de cellules (18, 18a, 18b) qui sont définies par les éléments de bordure (22) formés par le corps tubulaire,
une partie des cellules (18, 18a) est réalisée de façon allongée par rapport aux cellules restantes (18b) en direction longitudinale du stent (10), afin de constituer une extrémité frontale chanfreinée (24) du stent (10), **caractérisé en ce que**
une partie au moins des cellules allongées (18, 18a) est agencée le long d'une ligne droite ou approximativement droite qui s'étend parallèlement ou approximativement parallèlement à la direction longitudinale (L).

2. Stent (10) selon la revendication 1,
**caractérisé en ce que**
les cellules allongées (18, 18a) sont susceptibles d'être divisées en plusieurs groupes, en particulier en neuf groupes, les cellules (18, 18a) de chaque groupe étant agencées chacune le long d'une ligne droite ou approximativement droite, les lignes s'étendant en particulier parallèlement ou approximativement parallèlement à la direction longitudinale (L).

3. Stent (10) selon la revendication 2,
**caractérisé en ce que**
les lignes s'étendent parallèlement ou approximativement parallèlement les unes aux autres.

4. Stent (10) selon la revendication 2 ou 3,
**caractérisé en ce que**
un nombre égal de cellules respectives (18, 18a, 18b) est prévu dans chaque groupe depuis un plan de section transversale s'étendant perpendiculairement à l'axe longitudinal jusqu'à l'extrémité frontale chanfreinée (24).

5. Stent (10) selon l'une au moins des revendications 2 à 4,
**caractérisé en ce que**
la longueur des cellules (18, 18a, 18b) de groupes voisins diminue en direction périphérique pour passer d'un maximum à un minimum.

6. Stent (10) selon l'une au moins des revendications précédentes,
**caractérisé en ce que**
des cellules de longueur maximale (18a) sont en regard de cellules de longueur minimale (18b) par rapport à un axe central du stent (10).

7. Stent (10) selon l'une au moins des revendications précédentes,
**caractérisé en ce que**
les cellules (18, 18a, 18b) sont reliées entre elles au moins en partie au moyen de portions de liaison (20, 20a), et les portions de liaison (20a) entre les cellules allongées (18, 18a), en particulier uniquement entre les cellules (18a) les plus longues, sont réalisées de façon allongée.

8. Stent (10) selon l'une au moins des revendications précédentes,
**caractérisé en ce que**
au moins un marqueur (28, 28a), en particulier sous la forme d'un oeillet, s'étend en direction longitudinale (L) en éloignement de l'extrémité chanfreinée (24), le marqueur (28, 28a) présentant une forme asymétrique.

9. Stent (10) selon la revendication 8,
**caractérisé en ce que**
le marqueur (28, 28a) est agencé dans une zone entre l'extension la plus longue et l'extension la plus courte du stent (10) en direction longitudinale (L).

10. Stent (10) selon la revendication 8 ou 9,
**caractérisé en ce que**
il est prévu au moins deux marqueurs (28, 28a) à l'extrémité chanfreinée, les marqueurs (28, 28a) se faisant face par rapport à un axe du stent (10).

11. Stent (10) selon la revendication 1, qui est coupé à partir d'un matériau tubulaire et dans lequel, dans l'état expansé, la forme des cellules (18, 18a, 18b) est fixable.

12. Stent selon la revendication 11,
**caractérisé en ce que**
les cellules (18, 18a, 18b) du stent (10) sont fixables durablement dans la forme modifiée au moyen d'un processus thermique.
